# FASCICULE DE BREVET EUROPEEN

(11) **EP 2 755 650 B1**
(45) Date de publication et mention de la délivrance du brevet: **07.10.2015**
(21) Numéro de dépôt: 12759678.1
(22) Date de dépôt: 12.09.2012
(51) Int. Cl.: A61K 31/39, A61P 35/00, A61P 35/04

(54) **UTILISATION DE LA 3-(R)-[3-(2-METHOXYPHENYLTHIO)-2-(S)-METHYL-PROPYL]AMINO-3,4-DIHYDRO-2H-1,5-BENZOXATHIEPINE POUR LE TRAITEMENT DU CANCER ET EN PARTICULIER POUR LA PREVENTION ET/OU LE TRAITEMENT DES METASTASES CANCEREUSES**
VERWENDUNG VON 3-(R)-[3-(2-METHOXYPHENYLTHIO) 2 (S) -METHYLPROPYL-] AMINO-3,4-DIHYDRO 2H-1,5-BENZOXATHIEPIN ZUR BEHANDLUNG VON KREBS UND INSBESONDERE ZUR VORBEUGUNG UND/ODER BEHANDLUNG VON METASTASEN
USE OF 3-(R)-[3-(2-METHOXYPHENYLTHIO)-2-(S)-METHYLPROPYL]AMINO-3,4-DIHYDRO- 2H-1,5-BENZOXATHIEPINE FOR TREATING CANCER AND IN PARTICULAR FOR PREVENTING AND/OR TREATING CANCER METASTASES

(30) Priorité: 13.09.2011 FR 1158148
(43) Date de publication de la demande: 23.07.2014
(73) Titulaire: Pierre Fabre Médicament, 92100 Boulogne-Billancourt (FR)
(72) Inventeur: VACHER, Bernard, 81100 Castres (FR); LE GRAND, Bruno, 81220 Teyssode (FR)
(74) Mandataire: Regimbeau
(86) Numéro de dépôt international: PCT/EP2012/067780
(87) Numéro de publication internationale: WO 2013/037800

(56) Documents cités:
- WO-A1-02/081464
- WO-A1-2012/049439
- LE GRAND BRUNO ET AL: "Sodium late current blockers in ischemia reperfusion: is the bullet magic?", JOURNAL OF MEDICINAL CHEMISTRY 10 JUL 2008 LNKD- PUBMED:18529044, vol. 51, no. 13, 10 juillet 2008 (2008-07-10), pages 3856-3866, XP002673569, ISSN: 1520-4804
- BOCQUET A ET AL: "F 15845, a new blocker of the persistent sodium current prevents consequences of hypoxia in rat femoral artery.", BRITISH JOURNAL OF PHARMACOLOGY SEP 2010 LNKD- PUBMED:20735424, vol. 161, no. 2, septembre 2010 (2010-09), pages 405-415, XP002673570, ISSN: 1476-5381
- ONKAL RUSTEM ET AL: "Molecular pharmacology of voltage-gated sodium channel expression in metastatic disease: clinical potential of neonatal Nav1.5 in breast cancer.", EUROPEAN JOURNAL OF PHARMACOLOGY 25 DEC 2009 LNKD- PUBMED:19835862, vol. 625, no. 1-3, 25 décembre 2009 (2009-12-25), pages 206-219, XP002673571, ISSN: 1879-0712

## Description

L'invention concerne l'utilisation de la 3-(R)-[3-(2-méthoxyphénylthio)-2-(S)-méthyl-propyl]amino-3,4-dihydro-2H-1,5-benzoxathiépine ou l'un de ses sels pharmaceutiquement acceptables dans le traitement du cancer et en particulier dans la prévention et/ou le traitement des métastases cancéreuses.

La 3-(R)-[3-(2-méthoxyphénylthio)-2-(S)-méthyl-propyl] amino-3,4-dihydro-2H-1,5-benzoxathiépine représentée par la formule : 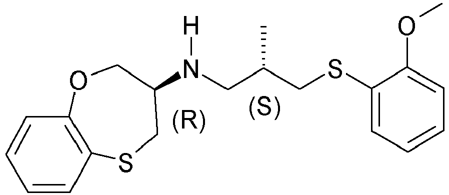 ses sels pharmaceutiquement acceptables ainsi que son utilisation dans le traitement de l'angor, de l'insuffisance cardiaque, de l'infarctus du myocarde et des troubles du rythme cardiaque sont décrit dans la demande de brevet WO 02/081464.

Le cancer peut être défini de façon très large comme une maladie liée à la prolifération et la diffusion incontrôlée de cellules de l'organisme devenues anormales. C'est l'une des premières causes de mortalité dans les pays développés et le nombre de nouveaux cas est en constante augmentation. Cependant, grâce entre autres aux progrès des traitements anticancéreux le taux de mortalité par cancer a significativement diminué. Les traitements anticancéreux incluent, suivant le type et le degré d'évolution de la maladie, la chirurgie, la radiothérapie et la chimiothérapie. Dans la plupart des cas, une combinaison de deux ou trois approches est nécessaire.

La radiothérapie est une méthode de traitement locorégional des cancers, utilisant des radiations ionisantes pour détruire les cellules cancéreuses tout en épargnant autant que possible les tissus sains voisins. La chimiothérapie consiste en l'utilisation de substances susceptibles de tuer ou de limiter la prolifération des cellules cancéreuses.

Lorsqu'un cancer est détecté à un stade précoce, c'est-à-dire avant qu'il n'y ait de métastases, le pronostic est relativement bon. Dans la pratique, cela ne représente cependant qu'environ 30% des cas. La tumeur, dite tumeur primaire, est alors généralement traitée localement par chirurgie et/ou par radiothérapie ; les patients pouvant recevoir une chimiothérapie complémentaire visant à diminuer les risques de récidives et d'apparition de tumeurs secondaires, ou métastases.

De nombreux type de cancers ont la capacité de former des métastases qui peuvent être distantes de la tumeur primaire et peuvent survenir plusieurs années après le traitement d'une tumeur primaire. Un phénomène que l'on explique par l'existence de micrométastases pré-angiogéniques ou de cellules qui restent sans se diviser pendant une durée prolongée au niveau du/des site(s) secondaire(s). En cancérologie, on entend par métastases, des tumeurs secondaires qui se sont formées par essaimage de cellules d'une tumeur primaire identifiée ou non. On parle de micrométastases lorsque la taille de ces tumeurs secondaires ne dépasse pas 2 mm.

Bien que les cellules métastatiques soient originaires de la tumeur primaire, elles ne sont pas exactement identiques aux cellules de cette dernière. En effet, ces cellules doivent acquérir un certain nombre de caractères leur permettant de passer du phénotype cancéreux à métastatique. Or, la formation des métastases est un phénomène complexe, abrégé ici « processus métastatique », au cours duquel des cellules cancéreuses quittent la tumeur primaire et migrent vers d'autres parties du corps en utilisant le système lymphatique et/ou sanguin. Le processus métastatique peut se décomposer en plusieurs étapes : 1) les cellules cancéreuses se détachent de la tumeur primaire et se lient en les dégradant aux protéines qui composent la matrice extracellulaire séparant la tumeur des tissus voisins, 2) une fois la matrice rompue, elles infiltrent les tissus environnants y compris les vaisseaux lymphatiques et/ou sanguins, 3) elles survivent alors dans la circulation et sont transportées jusqu'au niveau du/des site(s) secondaire(s) où elles sortent par extravasation, 4) elles se fixent dans un tissu et prolifèrent après activation du microenvironnement et induction de l'angiogenèse pour donner une métastase.

On entend par angiogenèse, l'ensemble des processus qui aboutissent à la formation de nouveaux capillaires sanguins à partir du réseau vasculaire préexistant.

Dans la plupart des cas de cancers, la mortalité n'est pas due à la tumeur primaire mais aux métastases qui se développent et se multiplient au niveau d'un ou plusieurs organes.

Le traitement des cancers métastatiques est basé essentiellement sur la chimiothérapie ; la radiothérapie et/ou la chirurgie intervenant le plus souvent en complément pour soulager certains symptômes. Dans la pratique, les traitements chimiothérapeutiques systémiques sont néanmoins peu efficaces sur les métastases et n'ont pas d'effet sur le processus métastatique lui-même.

Une autre stratégie a été développée qui consiste non pas à détruire les métastases mais plutôt à empêcher leur croissance au-delà de quelques mm en freinant l'angiogenèse tumorale au moyen de substances qui bloquent la prolifération des cellules de l'endothélium vasculaire.

Parmi les substances anti-angiogéniques utilisées dans les traitements anticancéreux on trouve le bevacizumab, le sorafenib et le sunitinib. Le bevacizumab est un anticorps monoclonal qui se lie à toutes les isoformes du facteur de croissance vasculaire (VEGF) et empêche sa liaison aux récepteurs du VEGF. Le sorafenib et le sunitinib sont des inhibiteurs non-sélectifs des récepteurs à tyrosine kinase, en particulier ceux du VEGF. Les substances anti-angiogéniques sont dotées d'une activité anti-métastatique indirecte puisqu'elles améliorent la délivrance des agents chimiothérapeutiques bien qu'elles n'interfèrent pas avec le processus métastatique lui-même. Lesdits médicaments présentent néanmoins de sérieux inconvénients : 1) ils diminuent la densité des micro-vaisseaux tissulaires et interfèrent avec les processus normaux de réparations ; 2) ils aggravent les maladies coronariennes, causent des hypertensions artérielles et des thromboses ; 3) il n'existe pas de facteurs prédictifs de la réponse ni de l'apparition de résistance, et 4) leur coût est très élevé.

Compte tenu des limites des traitements actuels, il existe un réel besoin médical pour des substances ayant la capacité de prévenir les métastases chez les patients souffrants d'un cancer. De plus, il apparaît fortement souhaitable que les substances en question possèdent des propriétés anti-métastatiques directes donc complémentaires de celles des médicaments existants.

Les canaux ioniques ont été décrits comme intervenant dans les mécanismes d'invasion et de migration nécessaires à la formation des métastases (Arcangeli et al. 2009, Curr. Med. Chem. 16, 66-93). Parmi les canaux anormalement exprimés dans les cellules cancéreuses, on retrouve le canal sodique voltage-dépendant Nav1.5 (Onkal and Djamgoz 2009, Eur. J. Pharmacol. 625, 206-219). Dans la présente invention, le terme « Nav1.5 » désigne les canaux sodiques voltage dépendants dont la sous-unité alpha qui constitue le pore du canal est codée par le gène SNC5A (dénommé aussi H1) situé sur le chromosome 3. La sous-unité alpha peut être associée à une ou plusieurs sous-unités auxiliaires (dénommée(s) sous-unité béta) codée(s) par les gènes SNC1B, SNC2B, SNC3B, SNC4B localisés sur les chromosomes 11 et 19.

Ainsi, il a été montré que l'expression de canaux Nav1.5 fonctionnels dans les cellules du cancer de l'ovaire augmente fortement leur potentiel métastatique (Gao et al. 2010, Oncology Reports 23, 1293-1299). De même, des canaux Nav1.5 fonctionnels ont été mis en évidence dans des biopsies de cancers du sein (Brisson et al. 2011, Oncogene 30, 2070-2076) ou de cancers du colon (House et al. 2010, Cancer Res. 70, 6957-6967) hautement métastatiques alors que les cellules saines des tissus correspondants ou celles de tumeurs non ou faiblement métastatiques n'en comportent pas. Des canaux Nav1.5 on été aussi détectés dans plusieurs lignées tumorales et biopsies de cancers du poumon (Roger et al. 2007, Int. J. Biochem. Cell Biol. 39, 774-786). L'expression ou la surexpression des canaux du type Nav1.5 a d'ailleurs été proposée comme outil de diagnostic du potentiel métastatique des cellules cancéreuses (Fraser et al. 2005, Clin. Cancer Res. 11, 5381-5389). Il a été aussi suggéré que l'effet bénéfique des acides gras insaturés à longues chaînes dans les cancers du sein, du colon et de la prostate pourrait être lié à leur propriétés inhibitrice du courant sodique Nav1.5, bien que leur mécanisme d'action ne soit pas connu (Gillet et al. 2011, Biochimie 93, 4-6). Des expériences *in vitro* indiquent que des bloqueurs du canal Nav1.5 tels que la tétrodotoxine ou des anticorps polyclonaux dirigés contre la forme néonatale du canal Nav1.5 atténuent le potentiel métastatique dans plusieurs lignées de cellules cancéreuses (Chioni et al. 2005, J. Neurosci. Methods 147, 88-98).

Un faisceau d'éléments indique donc que le courant sodique produit par le canal Nav1.5 joue un rôle majeur dans le processus métastatique des cellules cancéreuses, au moins dans certains types de cancers, en particulier les cancers du sein, du poumon, de la prostate, du colon, de la vessie, de l'ovaire, des testicules, de la peau, de la tyroïde ou d'un cancer gastrique. Les mécanismes par lesquels les canaux Nav1.5 potentialisent la formation de métastases n'ont pas été élucidés mais plusieurs hypothèses ont été avancées comme, par exemple, la modification de l'adhésion, la régulation des enzymes protéolytiques.Au final, le canal Nav1.5 apparaît donc comme une cible potentielle pour prévenir la formation des métastases.

Compte tenu du rôle ubiquitaire du canal Nav1.5, son exploitation en tant que cible thérapeutique pour la mise au point d'un agent anticancéreux et/ou anti-métastatique est néanmoins compliquée. En effet, le canal Nav1.5 est largement distribué dans l'organisme notamment au niveau des cellules cardiaques et vasculaires (Goldin 2001, Annu. Rev. Physiol. 63, 871-894).

Dans les cardiomyocytes, l'ouverture du canal Nav1.5 déclenche un courant sodique entrant qui peut se désactiver selon deux modes au moins, chacun caractérisé par la cinétique de fermeture du canal : l'inactivation rapide et l'inactivation lente. L'inactivation rapide engendre le courant Nav1.5 dit « rapide » qui ne dure que quelques millisecondes, alors que l'inactivation lente produit le courant dit « lent » qui dure lui plusieurs dizaines de millisecondes. Le courant Nav1.5 rapide joue un rôle fondamental dans le fonctionnement normal du coeur où il déclenche et assure la propagation du potentiel d'action cardiaque. Au contraire, celui du courant lent ne semble pas important dans le fonctionnement normal du coeur et n'est produit ou fortement amplifié que dans les cellules cardiaques et vasculaires soumises à un stress (Bocquet et al. 2010, Br. J. Pharmacol. 161, 405-415).

De manière surprenante, les inventeurs ont montré que la 3-(R)-[3-(2-méthoxyphénylthio)-2-(S)-méthyl-propyl]amino-3,4-dihydro-2H-1,5-benzoxathiépine qui est un inhibiteur sélectif du courant Nav1.5 lent, bloque également le courant sodique produit par les canaux sodiques Nav1.5 présents dans les cellules cancéreuses, abrégé ici « courant Nav1.5 métastatique ».

Sur la base de cette observation, il ressort que le courant Nav1.5 métastatique et le courant Nav1.5 lent ont des caractéristiques biophysiques suffisamment proches pour être tous les deux reconnus par la 3-(R)-[3-(2-méthoxyphénylthio)-2-(S)-méthyl-propyl]amino-3,4-dihydro-2H-1,5-benzoxathiépine.

Il ressort aussi que le courant Nav1.5 métastatique et le courant Nav1.5 rapide cardiaque sont en fait dissociables. En effet, les inventeurs ont montré que la 3-(R)-[3-(2-méthoxyphénylthio)-2-(S)-méthyl-propyl]amino-3,4-dihydro-2H-1,5-benzoxathiépine n'affecte pas le fonctionnement normal du système cardiovasculaire sous le contrôle du courant Nav1.5 rapide, et cela même aux fortes doses. La 3-(R)-[3-(2-méthoxyphénylthio)-2-(S)-méthyl-propyl]amino-3,4-dihydro-2H-1,5-benzoxathiépine possède donc la sélectivité d'action requise pour son utilisation en tant qu'agent anti-métastatique puisqu'il bloque le courant Nav1.5 métastatique sans interférer avec le courant Nav1.5 rapide indispensable au fonctionnement normal du coeur et des vaisseaux.

Parmi les inhibiteurs/bloqueurs des canaux Nav1.5, on trouve de nombreux composés non-Nav1.5 sélectifs tels que des toxines naturelles, des molécules thérapeutiques (e.g. agents anesthésiants, anti-arythmiques) et des insecticides (Anger et al. 2001, J. Med. Chem. 44, 115-137). Seuls deux médicaments sont décrits comme des bloqueurs préférentiels du courant Nav1.5 lent cardiaque : la ranolazine (RN 95635-55-5) et le riluzole (RN 1744-22-5), mais ils sont peu puissants et peu sélectifs vis à vis dudit courant. De plus, ils interagissent avec d'autres cibles moléculaires que le canal Nav1.5. De manière intéressante, le riluzole a été rapporté comme ayant une activité anti-métastatique dans les mélanomes, toutefois cette activité met en jeu d'autres mécanismes que l'inhibition du courant sodique Nav1.5 lent (US 20100221246 ; Biechele et al. 2010, Chemistry & Biology 17, 1177- 1182; Wu et al. 2009, NeuroToxicology 30, 677-685).

La présente invention fournit donc un nouveau moyen de combattre le cancer et plus particulièrement d'en prévenir ou traiter les métastases en agissant directement au niveau du processus métastatique, ce qu'aucun agent existant n'est capable de réaliser.

Bien que le canal Nav1.5 ait été identifié dans divers types de cancers métastatiques comme ceux du sein, du poumon, de la prostate, du colon, il est bien entendu que l'utilisation de la 3-(R)-[3-(2-méthoxyphénylthio)-2-(S)-méthyl-propyl]amino-3,4-dihydro-2H-1,5-benzoxathiépine ne se limite pas auxdits cancers mais s'étend à tous les cancers dont les cellules expriment, entre autres, le canal Nav1.5

Plus précisément et à titre d'exemple, il est montré selon l'invention que la 3-(R)-[3-(2-méthoxyphénylthio)-2-(S)-méthyl-propyl]amino-3,4-dihydro-2H-1,5-benzoxathiépine ou l'un de ses sels pharmaceutiquement acceptables bloque le courant Nav1.5 métastatique dans une lignée tumorale humaine de cancer mammaire fortement métastatique. Compte tenu du lien établit entre le courant produit par les canaux Nav1.5 présents dans les cellules cancéreuses et leur propension à former des métastases, la mise en évidence de propriétés inhibitrices dudit courant revient donc à révéler des propriétés anti-métastatiques. De plus, les inventeurs ont montré que la 3-(R)-[3-(2-méthoxyphénylthio)-2-(S)-méthyl-propyl]amino-3,4-dihydro-2H-1,5-benzoxathiépine n'était pas cytotoxique.

La présente invention a pour objet la 3-(R)-[3-(2-méthoxyphénylthio)-2-(S)-méthyl-propyl]amino-3,4-dihydro-2H-1,5-benzoxathiépine ou l'un de ses sels pharmaceutiquement acceptables pour son utilisation en tant que médicament destiné au traitement du cancer et plus particulièrement pour en prévenir ou traiter les métastases.

Dans la présente invention, le terme « pharmaceutiquement acceptable » se réfère à des entités moléculaires et des compositions qui ne produisent aucun effet adverse, allergique ou autre réaction indésirable quand elles ont administrées à un humain. Quand utilisé ici, le terme « excipient pharmaceutiquement acceptable » inclut tout diluant, adjuvant ou excipient, tels que des agents préservatifs, des agents de remplissage, des agents désintégrant, mouillant, émulsifiant, dispersant, antibactérien ou antifongique, ou bien encore des agents qui permettraient de retarder l'absorption et la résorption intestinale et digestive. L'utilisation de ces milieux ou vecteurs est bien connu de l'homme du métier.

On désigne par « sels pharmaceutiquement acceptables » d'un composé des sels qui sont pharmaceutiquement acceptables, comme défini ici et qui possèdent l'activité pharmacologique souhaitée du composé parent. De tels sels comprennent : les sels d'addition d'acide formés avec des acides minéraux tels que l'acide chlorhydrique, l'acide bromhydrique, l'acide sulfurique, l'acide nitrique, l'acide phosphorique et similaires ou formés avec des acides organiques tels que l'acide acétique, l'acide benzènesulfonique, l'acide benzoïque, l'acide camphresulfonique, l'acide citrique, l'acide éthane-sulfonique, l'acide fumarique, l'acide glucoheptonique, l'acide gluconique, l'acide glutamique, l'acide glycolique, l'acide hydroxynaphtoïque, l'acide 2-hydroxyéthanesulfonique, l'acide lactique, l'acide maléique, l'acide malique, l'acide mandélique, l'acide méthanesulfonique, l'acide muconique, l'acide 2-naphtalènesulfonique, l'acide propionique, l'acide salicylique, l'acide succinique, l'acide dibenzoyl-L-tartrique, l'acide tartrique, l'acide p-toluènesulfonique, l'acide triméthylacétiq u e , l'acide trifluoroacétique et similaires.

Les sels pharmaceutiquement acceptables comprennent également les formes d'addition de solvants (solvates) ou les formes cristallines (polymorphes) tels que définis ici, du même sel d'addition d'acide.

L'invention a également pour objet l'utilisation de la 3-(R)-[3-(2-méthoxyphénylthio)-2-(S)-méthyl-propyl]amino-3,4-dihydro-2H-1,5-benzoxathiépine ou l'un de ses sels pharmaceutiquement acceptables, chez des patients présentant une/des tumeur(s) cancéreuse(s) dont les cellules expriment, entre autres, le canal sodique voltage-dépendant Nav1.5.

La présente invention concerne en outre une composition pharmaceutique contenant comme agent actif la 3-(R)-[3-(2-méthoxyphénylthio)-2-(S)-méthyl-propyl]amino-3,4-dihydro-2H-1,5-benzoxathiépine ou l'un de ses sels pharmaceutiquement acceptables et au moins un excipient pharmaceutiquement acceptable, pour son utilisation dans le traitement du cancer et plus particulièrement pour prévenir ou traiter les métastases cancéreuses. D'une façon préférentielle, les cancers concernés par la composition de la présente invention sont ceux : du sein, du poumon, de la prostate, du colon, de la vessie, de l'ovaire, des testicules, de la peau, de la tyroïde ou gastrique.

D'une façon préférée, la composition pharmaceutique selon l'invention s'adresse à des patients dont les cellules tumorales expriment, entre autres, le canal Nav1.5. La présence dudit canal dans les cellules tumorales du patient peut être révélée par la présence de l'ARN messager du gène SCN5A et/ou de la protéine canal elle-même. L'ARN messager et/ou la protéine peuvent être mis en évidence au moyen de techniques bien connues de l'homme du métier telles que, par exemple, la PCR (polymerase chain reaction), le Western blot ou l'hybridation *in situ.* Les cellules peuvent être obtenues à partir de prélèvements effectués au niveau de la tumeur primaire, de métastases, de ganglions lymphatiques ou sanguins et être analysées directement ou être mise en culture *in vitro* avant d'être analysées.

La composition pharmaceutique selon l'invention, peut être administrée avec un ou d'autre(s) agent(s) actif(s), tel qu'un agent anticancéreux, ou en association avec un traitement radiothérapeutique ou chirurgical, ou avec une combinaison de ces derniers. L'administration peut être alors simultanée, séparée ou décalée par rapport à/aux (l')autre(s) traitement(s). Elle peut également être utilisée pendant toute la durée ou sur une durée plus courte ou plus longue que celle de l'autre traitement anticancéreux.

Les compositions pharmaceutiques selon la présente invention sont formulées pour l'administration à l'être humain. Les compositions selon l'invention peuvent être administrées par voie orale, sublinguale, sous-cutanée, intramusculaire, intraveineuse, transdermique, locale ou rectale ou bien encore intra-nasale. Dans ce cas le principe actif peut être administré sous formes unitaires d'administration, en mélange avec des supports pharmaceutiques classiques, aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée ou transdermique, topique, intramusculaire, intraveineuse, intra-nasale ou intraoculaires, les formes d'administration rectale.

Lorsque l'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique, la silice ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, un antiseptique, ainsi qu'un agent donnant du goût et un colorant approprié.

Les poudres ou les granules dispersibles dans l'eau peuvent contenir l'ingrédient actif en mélange avec des agents de dispersion ou des agents mouillant, ou des agents de mise en suspension, de même qu'avec des correcteurs du goût ou des édulcorants.

Pour une administration rectale, on recourt à des suppositoires qui sont préparés avec des liants fondant à la température rectale, par exemple du beurre de cacao ou des polyéthylènes glycols.

Pour une administration parentérale (intraveineuse, intramusculaire, intradermique, sous-cutanée), intra-nasale ou intraoculaire, on utilise des suspensions aqueuses, des solutions salines isotoniques ou des solutions stériles et injectables qui contiennent des agents de dispersion et/ou des agents mouillants pharmacologiquement compatibles.

Le principe actif peut également être formulé sous forme de microcapsules, éventuellement avec un ou plusieurs supports additifs.

Avantageusement, la composition pharmaceutique selon la présente invention est destinée à une administration par voie orale ou intraveineuse.

La composition pharmaceutique selon la présente invention peut comprendre d'autres principes actifs conduisant à un effet complémentaire ou éventuellement synergique.

Les dosages de la 3-(R)-[3-(2-méthoxyphénylthio)-2-(S)-méthyl-propyl]amino-3,4-dihydro-2H-1,5-benzoxathiépine ou l'un de ses sels pharmaceutiquement acceptables dans les compositions de l'invention peuvent être ajustés afin d'obtenir une quantité de substance qui est efficace pour obtenir la réponse thérapeutique désirée pour une composition particulière à la méthode d'administration. La dose efficace du composé de l'invention varie en fonction de nombreux paramètres tels que, par exemple, la voie d'administration choisie, le poids, l'âge, le sexe et la sensibilité de l'individu à traiter. En conséquence, la posologie optimale devra être déterminée par le spécialiste en la matière en fonction des paramètres qu'il juge pertinents. Bien que les doses efficaces puissent varier dans de larges proportions, les doses journalières pourraient s'échelonner entre 1 mg et 1000 mg par 24 heures, et préférentiellement entre 1 et 200 mg, pour un adulte d'un poids moyen de 70 kg, en une ou plusieurs prises.

L'exemple suivant permet de mieux comprendre l'invention.

A titre purement illustratif les inventeurs ont choisi d'utiliser dans l'expérience la lignée MDA-MB-231 qui est une lignée d'adénocarcinome mammaire humain fortement métastatique. En effet, il a été montré que lesdites cellules expriment entre autres des canaux sodiques voltages-dépendants Nav1.5 fonctionnels et que le blocage dudit canal au moyen de divers outils pharmacologiques réduisait leur potentiel métastatique (Brackenbury et al. 2007, Breast Cancer Res. Treat. 101, 149-160). Cependant, les outils pharmacologiques en question ne permettent pas de caractériser de façon précise la nature du courant sodique Nav1.5 impliqué dans le processus métastatique. Or les inventeurs ont montré par des expériences de patch-clamp en configuration « cellule entière », réalisées sur des cellules MDA-MB-231, que la 3-(R)-[3-(2-méthoxyphénylthio)-2-(S)-méthyl-propyl]amino-3,4-dihydro-2H-1,5-benzoxathiépine réduisait de façon concentration-dépendante le courant Nav1.5 métastatique (IC₅₀ = 1.5 µM). De ce fait, les inventeurs considèrent que la 3-(R)-[3-(2-méthoxyphénylthio)-2-(S)-méthyl-propyl]amino-3,4-dihydro-2H-1,5-benzoxathiépine est doté de propriétés anti-métastatiques vis-à-vis des cellules cancéreuses qui expriment le canal Nav1.5. A ce titre, il est important de noter que de nombreux types de cancers métastatiques ont été rapportés comme exprimant le canal Nav1.5.

### Méthode

Culture cellulaire : les cellules MDA-MB-231 sont cultivées dans un milieu de culture de type Eagle, modifié par Dulbecco (Life Technologies LTD, Paisley, UK) supplémenté par de la L-glutamine 4 mM et 10% de sérum de veau foetal. Les cellules sont ensemencées dans des boites de culture de 100 mm et placées dans un incubateur à 37°C, 100% d'humidité et 5% de CO₂.

Mesures électrophysiologiques en configuration « cellule entière » : la pipette de patch (résistance 5-15 MΩ) contient une solution de : NaCl 5 mM, CsCl 145 mM, MgCl₂ 2 mM, CaCl₂ 1 mM, HEPES 10 mM et EGTA 11 mM, le pH est ajusté à 7,4 avec CsOH. L'électrode de référence est plongée dans le milieu extracellulaire constitué d'une solution de : NaCl 140 mM, KCl 4 mM, MgCl₂ 2 mM, glucose 11 mM, HEPES 10 mM, le pH est ajusté à 7,4 avec NaOH. Ces deux électrodes sont connectées à un amplificateur Axopatch 200B (Axon Instrument). Les courants sont filtrés au moyen d'un filtre Bessel à une fréquence de 5 kHz et sont échantillonnés à une fréquence de 5 kHz au moyen de l'interface Digidata (1200). L'acquisition et l'analyse des données sont réalisées au moyen du logiciel pClamp (Axon Instrument). Le potentiel de maintien est fixé à -110 mV pour enregistrer l'activité maximale des canaux Nav.

Deux protocoles ont été utilisés :
1 / Protocole « courant I_{Na} en fonction du voltage » permettant d'observer l'amplitude maximale de I_{Na} en fonction du voltage imposé. Des dépolarisations par paliers de 5 mV sont réalisées à une fréquence de 0,2 Hz, de -110 à +60 mV. Le créneau de dépolarisation dure 600 ms (cf. Figure annexée).
2/ Protocole de « dépolarisations répétées » qui permet de mesurer l'effet du produit test sur l'amplitude du courant. Pour cela, des dépolarisations à -20 mV se succèdent à une fréquence de 0,5 Hz. Le créneau de dépolarisation dure 25 ms.

### Résultats

Les cellules testées expriment un courant d'amplitude maximale de l'ordre de 900 pA. Le seuil d'activation du courant se situe à -50 mV, le pic de courant autour de -20 mV et le potentiel d'inversion à +30 mV (Figure annexée). Le courant est aboli en l'absence d'ions Na⁺ dans le milieu ce qui confirme que le courant en question est un courant sodique. L'inhibition du courant de pic par la tétrodotoxine (Sigma) n'est que partielle soit : 30 ± 7% à 1 µM et 54 ± 8% à 5 µM, ce qui indique que le courant en question est tétrodotoxine résistant.

Les inventeurs ont par ailleurs montré *in vivo* que la 3-(R)-[3-(2-méthoxyphénylthio)-2-(S)-méthyl-propyl]amino-3,4-dihydro-2H-1,5-benzoxathiépine n' interfère pas avec le fonctionnement normal du coeur même aux fortes doses.

La présente invention se distingue donc: 1) par le fait que la 3-(R)-[3-(2-méthoxyphénylthio)-2-(S)-méthyl-propyl]amino-3,4-dihydro-2H-1,5-benzoxathiépine agit directement sur le processus métastatique et, à ce titre, est complémentaire aux traitements existants ; 2) par le fait que la 3-(R)-[3-(2-méthoxyphénylthio)-2-(S)-méthyl-propyl]amino-3,4-dihydro-2H-1,5-benzoxathiépine agit sélectivement au niveau des cellules cancéreuses sans interférer, aux doses anti-métastatiques, avec les autres fonctions dans lesquelles sont impliquées les canaux sodiques Nav1.5 comme le fonctionnement normal du coeur et des vaisseaux.

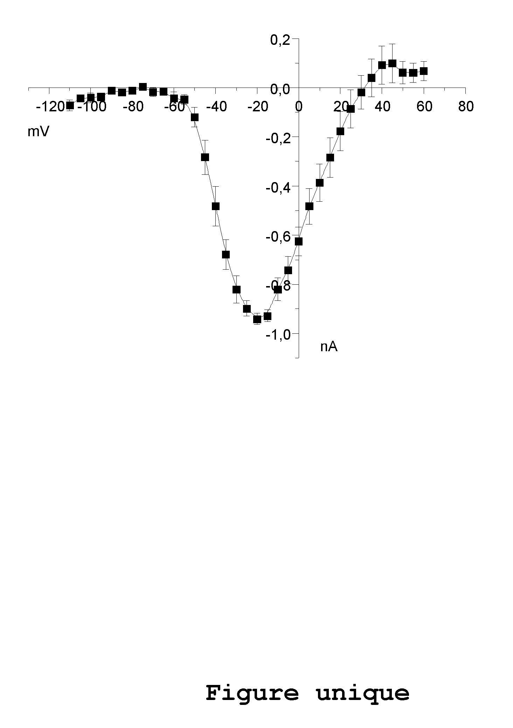

## Revendications

1. La 3-(R)-[3-(2-méthoxyphénylthio)-2-(S)-méthyl-propyl] amino-3,4-dihydro-2H-1,5-benzoxathiépine ou l'un de ses sels pharmaceutiquement acceptables, pour son utilisation en tant que médicament destiné au traitement du cancer.

2. La 3-(R)-[3-(2-méthoxyphénylthio)-2-(S)-méthyl-propyl] amino-3,4-dihydro-2H-1,5-benzoxathiépine ou l'un de ses sels pharmaceutiquement acceptables, pour son utilisation selon la revendication 1, pour prévenir ou traiter des métastases cancéreuses.

3. L a 3-(R)-[3-(2-méthoxyphénylthio)-2-(S)-méthyl-propyl] amino-3,4-dihydro-2H-1,5-benzoxathiépine ou l'un de ses sels pharmaceutiquement acceptables, pour son utilisation selon l'une des revendications 1 ou 2, chez des patients présentant une/des tumeur(s) cancéreuse(s) dont les cellules expriment, entre autres, le canal sodique voltage-dépendant Nav1.5.

4. La 3-(R)-[3-(2-méthoxyphénylthio)-2-(S)-méthyl-propyl] amino-3,4-dihydro-2H-1,5-benzoxathiépine ou l'un de ses sels pharmaceutiquement acceptables, pour son utilisation selon l'une quelconque des revendications 1 à 3, chez des patients atteints d'un cancer du sein, du poumon, de la prostate, du colon, de la vessie, de l'ovaire, des testicules, de la peau, de la tyroïde ou d'un cancer gastrique.

5. Composition pharmaceutique **caractérisée en ce qu'**elle contient comme agent actif la 3-(R)-[3-(2-méthoxyphénylthio)-2-(S)-méthyl-propyl]amino-3,4-dihydro-2H-1,5-benzoxathiépine ou l'un de ses sels pharmaceutiquement acceptables et au moins un excipient pharmaceutiquement acceptable, pour son utilisation en tant que médicament destiné au traitement du cancer.

6. Composition pharmaceutique selon la revendication 5, pour son utilisation afin de prévenir ou traiter des métastases cancéreuses.

7. Composition pharmaceutique selon l'une des revendications 5 ou 6, pour son utilisation chez des patients présentant une/des tumeur(s) cancéreuse(s) dont les cellules expriment, entre autres, le canal sodique voltage-dépendant Nav1.5.

8. Composition pharmaceutique selon l'une quelconque des revendications 5 à 7, pour son utilisation chez des patients atteints d'un cancer du sein, du poumon, de la prostate, du colon, de la vessie, de l'ovaire, des testicules, de la peau, de la tyroïde ou d'un cancer gastrique.

9. Composition pharmaceutique selon l'une quelconque des revendications 5 à 8, pour son utilisation chez des patients soumis à un traitement chimiothérapeutique.

10. Composition pharmaceutique selon l'une quelconque des revendications 5 à 9, pour son utilisation chez des patients soumis à un traitement radiothérapeutique et/ou chirurgical.

11. Composition pharmaceutique, selon l'une des revendications 9 ou 10, pour son utilisation simultanée, séparée ou décalée par rapport à/aux (l') autre(s) traitement(s)chimiothérapeutique(s), radiothérapeutique(s) et/ou chirurgical(aux).

12. Composition pharmaceutique selon l'une quelconque des revendications 5 à 11, pour son utilisation par voie orale ou intraveineuse.

13. Composition pharmaceutique, pour son utilisation selon l'une quelconque des revendications 5 à 12, **caractérisée en ce qu'**elle est présentée sous la forme d'une unité de dosage quotidienne de la 3-(R)-[3-(2-méthoxyphénylthio)-2-(S)-méthyl-propyl] amino-3,4-dihydro-2H-1,5-benzoxathiépine ou l'un de ses sels pharmaceutiquement acceptables comprise entre 1 et 1000 mg.

## Patentansprüche

1. 3-(R)-[3-(2-methoxyphenylthio)-2-(S)-methyl-propyl]amino-3,4-dihydro-2H-1,5-benzoxathiepin oder eines seiner pharmazeutisch akzeptablen Salze für seine Verwendung als Arzneimittel, das zur Behandlung des Krebses bestimmt ist.

2. 3-(R)-[3-(2-methoxyphenylthio)-2-(S)-methyl-propyl]amino-3,4-dihydro-2H-1,5-benzoxathiepin oder eines seiner pharmazeutisch akzeptablen Salze für seine Verwendung nach Anspruch 1 zur Vorbeugung oder Behandlung von kanzerogenen Metastasen.

3. 3-(R)-[3-(2-methoxyphenylthio)-2-(S)-methyl-propyl]amino-3,4-dihydro-2H-1,5-benzoxathiepin oder eines seiner pharmazeutisch akzeptablen Salze für seine Verwendung nach einem der Ansprüche 1 oder 2 bei Patienten, die einen Krebstumor/Krebstumoren aufweisen, dessen/deren Zellen unter anderem den spannungsabhängigen Natriumkanal Nav1.5 exprimieren.

4. 3-(R)-[3-(2-methoxyphenylthio)-2-(S)-methyl-propyl]amino-3,4-dihydro-2H-1,5-benzoxathiepin oder eines seiner pharmazeutisch akzeptablen Salze für seine Verwendung nach einem der Ansprüche 1 bis 3 bei Patienten, die unter Brust-, Lungen-, Prostata-, Darm-, Harnblasen-, Eierstock-, Hoden-, Haut-, Schilddrüsen- oder einem Magenkrebs leiden.

5. Pharmazeutische Zusammensetzung, **dadurch gekennzeichnet, dass** sie als Wirkstoff 3-(R)-[3-(2-methoxyphenylthio)-2-(S)-methyl-propyl]amino-3,4-dihydro-2H-1,5-benzoxathiepin oder eines seiner pharmazeutisch akzeptablen Salze und mindestens einen pharmazeutisch akzeptablen Hilfsstoff für seine Verwendung als Arzneimittel, das zur Behandlung des Krebses bestimmt ist, enthält.

6. Pharmazeutische Zusammensetzung nach Anspruch 5 für ihre Verwendung zur Vorbeugung oder Behandlung von kanzerogenen Metastasen.

7. Pharmazeutische Zusammensetzung nach einem der Ansprüche 5 oder 6 für ihre Verwendung bei Patienten, die einen Krebstumor/Krebstumoren aufweisen, dessen/deren Zellen unter anderem den spannungsabhängigen Natriumkanal Nav1.5 exprimieren.

8. Pharmazeutische Zusammensetzung nach einem der Ansprüche 5 bis 7 für ihre Verwendung bei Patienten, die unter Brust-, Lungen-, Prostata-, Darm-, Harnblasen-, Eierstock-, Hoden-, Haut-, Schilddrüsen- oder einem Magenkrebs leiden.

9. Pharmazeutische Zusammensetzung nach einem der Ansprüche 5 bis 8 für ihre Verwendung bei Patienten, die eine chemotherapeutische Behandlung absolvieren.

10. Pharmazeutische Zusammensetzung nach einem der Ansprüche 5 bis 9 für ihre Verwendung bei Patienten, die eine strahlentherapeutische und/oder chirurgische Behandlung absolvieren.

11. Pharmazeutische Zusammensetzung nach einem der Ansprüche 9 oder 10 für ihre gleichzeitige, getrennte oder versetzte Verwendung in Bezug auf die andere/n chemotherapeutische/n, strahlentherapeutische/n und/oder chirurgische/n Behandlung/en.

12. Pharmazeutische Zusammensetzung nach einem der Ansprüche 5 bis 11 für ihre Verwendung auf oralem oder intravenösem Weg.

13. Pharmazeutische Zusammensetzung für ihre Verwendung nach einem der Ansprüche 5 bis 12, **dadurch gekennzeichnet, dass** sie in Form einer täglichen Dosierungseinheit von 3-(R)-[3-(2-methoxyphenylthio)-2-(S)-methyl-propyl]amino-3,4-dihydro-2H-1,5-benzoxathiepin oder eines seiner pharmazeutisch akzeptablen Salze zwischen 1 und 1000 mg inklusive erfolgt.

## Claims

1. 3-(R)-[3-(2-methoxyphenylthio)-2-(S)-methyl-propyl] amino-3,4-dihydro-2H-1,5-benzoxathiepine or one of the pharmaceutically acceptable salts thereof, for use as a medicinal product for treating cancer.

2. 3-(R)-[3-(2-methoxyphenylthio)-2-(S)-methyl-propyl] amino-3,4-dihydro-2H-1,5-benzoxathiepine or one of the pharmaceutically acceptable salts thereof, for use according to claim 1, for preventing or treating cancer metastases.

3. 3-(R)-[3-(2-methoxyphenylthio)-2-(S)-methyl-propyl] amino-3,4-dihydro-2H-1,5-benzoxathiepine or one of the pharmaceutically acceptable salts thereof, for use according to one of claims 1 or 2, in patients presenting one or more cancerous tumours, the cells of which express, inter alia, the Nav1.5 voltage-gated sodium channel.

4. 3-(R)-[3-(2-methoxyphenylthio)-2-(S)-methyl-propyl] amino-3,4-dihydro-2H-1,5-benzoxathiepine or one of the pharmaceutically acceptable salts thereof, for use according to any one of claims 1 to 3, in patients suffering from breast, lung, prostate, colon, bladder, ovarian, testicular, skin, thyroid or stomach cancer.

5. Pharmaceutical composition **characterised in that** it contains, as an active agent, 3-(R)-[3-(2-methoxyphenylthio)-2-(S)-methyl-propyl]amino-3,4-dihydro-2H-1,5-benzoxathiepine or one of the pharmaceutically acceptable salts thereof and at least one pharmaceutically acceptable excipient, for use as a medicinal product for treating cancer.

6. Pharmaceutical composition according to claim 5, for use for preventing or treating cancer metastases.

7. Pharmaceutical composition according to one of claims 5 or 6, for use in patients presenting one or more cancerous tumours, the cells of which express, inter alia, the Nav1.5 voltage-gated sodium channel.

8. Pharmaceutical composition according to any one of claims 5 to 7, for use in patients suffering from breast, lung, prostate, colon, bladder, ovarian, testicular, skin, thyroid or stomach cancer.

9. Pharmaceutical composition according to any one of claims 5 to 8, for use in patients undergoing chemotherapy treatment.

10. Pharmaceutical composition according to any one of claims 5 to 9, for use in patients undergoing radiotherapy and/or surgical treatment.

11. Pharmaceutical composition, according to one of claims 9 or 10, for the simultaneous, separate or staggered use thereof in relation to the other chemotherapy, radiotherapy and/or surgical treatment(s).

12. Pharmaceutical composition according to any one of claims 5 to 11, for use thereof by oral or intravenous route.

13. Pharmaceutical composition for use according to any one of claims 5 to 12, **characterised in that** it is in the form of a daily dosage unit of 3-(R)-[3-(2-methoxyphenylthio)-2-(S)-methyl-propyl] amino-3,4-dihydro-2H-1,5-benzoxathiepine or one of the pharmaceutically acceptable salts thereof between 1 and 1000 mg.
